# DEMANDE DE BREVET EUROPEEN

(11) **EP 1 787 515 A2**
(43) Date de publication de la demande: **23.05.2007**
(21) Numéro de dépôt: 06291539.2
(22) Date de dépôt: 02.10.2006
(51) Int. Cl.: A01P 1/00, A01P 3/00, A01N 59/14, A43B 13/38

(54) **Composition antibactérienne et antifongique pour chaussures**

(30) Priorité: 21.11.2005 FR 0511827
(71) Demandeur: Dos Santos, Christophe, 76520 Gouy (FR)
(72) Inventeur: Dos Santos, Christophe, 76520 Gouy (FR)
(74) Mandataire: Wagret, Frédéric

(57) **Abrégé**

Composition antibactérienne et antifongique absorbant l'humidité des chaussures provenant de la transpiration des pieds, caractérisée en ce qu'elle est constituée d'au moins un biocide, comprenant des fonctions antiseptiques, bactériostatiques et fongistatiques sans interférence avec le processus physiologique normal de sudation. De préférence, ledit au moins biocide est un acide borique ou acide orthoborique ou acide boracique. Cette composition peut également contenir au moins un autre principe actif. Alternativement, la composition contient également au moins un agent absorbant et/ou adsorbant et/ou au moins une substance odorante.

## Description

La présente invention appartient aux domaines de la dermatologie et de la cosmétique.

L'invention concerne les compositions utilisées en podologie-pédicurie, ou en cosmétique, pour la prévention ou le traitement des mauvaises odeurs issues de la sudation des pieds.

La sudation est un processus physiologique normal et indispensable qui constitue une fonction principale de la thermorégulation du corps. La transpiration se traduit par l'évacuation de la sueur par les pores de la peau.

Les désagréments de la transpiration abondante proviennent de l'hyperstimulation des glandes sudoripares composées, d'une part, de glandes eccrines assurant la thermorégulation de notre corps, et d'autre part, de glandes apocrines, responsables des mauvaises odeurs.

En effet, les glandes eccrines, présentes sur la plupart des parties de notre peau, sont nombreuses au niveau des paumes des mains, des plantes des pieds, et du front. La sueur qu'elles libèrent est constituée d'eau salée hypotonique. Ces glandes provoquent l'évaporation de l'eau à la surface de la peau pour faire diminuer la température corporelle, ou afin par exemple, de répondre à une anxiété, un stress, un effort physique. La sueur provenant de ce type de glandes sudoripares est presque inodore.

Toutefois, une odeur corporelle peut apparaître, résultant de la transpiration avec l'activité des bactéries vivant naturellement à la surface de la peau. Ces bactéries prolifèrent dans la sueur et particulièrement dans celle provenant des glandes apocrines. Ces dernières ne participent pas au refroidissement du corps. Elles libèrent une sécrétion alcaline qui confère à la sueur un aspect trouble et une odeur spéciale. L'odeur corporelle provient donc d'un mélange complexe de substances chimiques formé de sébum, de sueur, de l'activité des bactéries sur la peau et des hormones (contenues dans le sébum et la sueur apocrine).

La transpiration est généralement considérée comme étant une simple manifestation d'un stress, d'une émotivité, d'un exercice physique important, d'une réponse à une température ambiante élevée.

Cependant, il peut se révéler que la transpiration puisse être pathologique. En effet, certaines personnes souffrent d'une exagération importante de la sécrétion sudorale appelée hyperhidrose occasionnant une sudation abondante. Cette sudation abondante consiste en des poussées de transpiration se déclenchant au niveau des aisselles, des paumes des mains, et des plantes des pieds, avec une intensité qui peut devenir invalidante voir une bromhidrose. Cette dernière est une affection qui se caractérise par une sécrétion sudorale plus ou moins abondante mais surtout nauséabonde.

Cette sudation excessive engendre, par exemple, un phénomène de macération dans les chaussures pouvant entraîner diverses pathologies, telles, que des mycoses, des infections bactériennes, des phlyctènes, dés dermites de contact, des verrues plantaires, des bromhidroses, des plages érythémateuses ou des kératolyses ponctuées sur les zones d'appui des plantes des pieds.

Les anti-cholinergiques tels que l'atropine ou la belladone, utilisés autrefois par voie orale ou en application locale ont été écartés du fait de leur efficacité à doses toxiques et de leur effets secondaires indésirables.

Afin de supprimer les mauvaises odeurs, les méthodes actuelles consistent soit à :
- masquer l'odeur par des parfums : les déodorants ou désodorisant,
- supprimer la flore bactérienne locale : les antiseptiques,
- limiter l'excès de production de sueur : les anti-transpirants.

Les substances utilisées comme « masques » des mauvaises odeurs sont généralement des substances tampons, telles que, le propylène glycol ou le stéarate de sodium. Il est aussi généralement appliqué des parfums puissants sur les pieds et les chaussures. L'effet souhaité de ces produits n'est pas durable et, souvent, l'odeur résultante de l'association du parfum et des pieds est désagréable.

Les résultats obtenus avec ces substances « masquantes » demeurent donc aléatoires et temporaires.

Afin d'éradiquer les bactéries, il est nécessaire d'employer des antiseptiques locaux. Certains présentant une toxicité trop importante ne sont plus utilisés et d'autres sont utilisés en association avec d'autres produits. Par exemple, le triclocarban ou le triclosan sont associés à d'autres substances, telles que des savons ou d'autres bases nettoyantes. Ces produits ne donnent pas entière satisfaction quant aux conséquences résultantes de l'hyperproduction de sueur, et demandent une fréquence d'hygiène multi-quotidienne.

Il existe également des substances provoquant le blocage de la sécrétion-excrétion sudorale. Ces « bloqueurs » de l'excrétion sudorale obstruent temporairement les canaux sudoraux et ne sont donc efficaces que lors de leur utilisation.

Les anti-transpirants, tels que, les sels de brome et de zirconium ne sont également plus employés, étant donné qu'ils sont responsables de la formation de granulomes toxiques à corps étrangers.

Le brevet américain, n° US 2006/045915, divulgue une composition en poudre pour éliminer des odeurs fétides liées à la bromhidrose et à l'hyperhidrose. La composition inclut du sulfate double d'aluminium et de potassium, de l'oxyde de zinc et de l'undécylénate de zinc. Cette composition en poudre est employée dans des chaussures et sur des semelles, afin d'éliminer les bactéries et d'empêcher la prolifération bactérienne.

Les sels d'aluminium sont des produits de référence pour leurs effets antisudoraux reconnus. En effet, en présence d'eau, l'aluminium donne des hydroxydes avec acidification du milieu, d'où une réduction de l'humidité locale, de la flore bactérienne et de la bromhidrose. Toutefois, ces sels occasionnent une acidité irritante au contact de la peau et des textiles fragiles constituant les chaussures.

Actuellement, il existe également des semelles de chaussures contenant du charbon actif absorbant une partie de l'humidité en excès. Cependant, une fois le matériau saturé, cette dernière reste prisonnière à l'intérieur des chaussures.

Ces substances connues sont généralement conditionnées sous forme de nettoyant, de gel, de crème, de spray, de vaporisateur, de lotion, de stick à appliquer sur la peau et/ou les chaussures.

Elles possèdent comme principale fonction de dissimuler des odeurs issues de la transpiration, et de supprimer la flore bactérienne voire d'empêcher la transpiration.

La présente invention remédie aux inconvénients de l'art antérieur en proposant une composition permettant de lutter contre les conséquences et les mauvaises odeurs résultantes de la transpiration, sans altérer ou interférer son processus physiologique normal.

Plus particulièrement, la composition, selon l'invention, absorbe l'humidité notamment des chaussures, des semelles ou du textile formant lesdites chaussures, permettant ainsi de limiter la macération et la prolifération des bactéries, des champignons et autres, et donc de désodoriser sans porter atteinte à la flore bactérienne naturelle de la peau.

De manière avantageuse, cette composition asséchante n'engendre pas la suppression de la sudation tout en ayant des fonctions antiseptiques, bactériostatiques et fongistatiques.

La composition, selon l'invention, permet de pallier à la macération et à l'accumulation de bactéries issues de la transpiration dans les chaussures provocant une odeur désagréable. De ce fait, la composition désodorisante, selon l'invention, ne masque pas simplement l'odeur. Au contraire, elle agit contre la prolifération des bactéries.

A cette fin, la composition selon l'invention est une formule antibactérienne et antifongique absorbant l'humidité des chaussures provenant de la transpiration des pieds, caractérisée en ce qu'elle est constituée d'au moins un biocide comprenant des fonctions antiseptiques, bactériostatiques et fongistatiques sans interférence avec le processus physiologique normal de sudation.

De préférence, ledit au moins un biocide est un acide borique ou acide orthoborique ou acide boracique.

Cette composition peut également contenir au moins un autre principe actif.

Il peut être prévu que ladite composition comprenne au moins un agent absorbant et/ou adsorbant et/ou au moins une substance odorante.

De manière préférentielle, la composition selon l'invention est constituée d'environ de 40 à 98% d'acide borique, de 1.5 à 5.5% de kaolin, de 0.2 à 0.5% de lavande, de 0.05 à 0.2% de sauge et environ de 0.05 % à 0.2 % d'argile.

La composition selon l'invention peut être soit sous forme de poudre, conditionnée dans une boîte saupoudreuse ou dans un sachet par exemple ; soit sous forme de gel ou de crème ou de spray ou de vaporisateur ou de lotion ou de stick ou de semelle.

La présente invention est maintenant décrite à l'aide d'exemples uniquement illustratifs, et nullement limitatifs de la portée de la présente invention.

La composition selon l'invention est destinée à supprimer les conséquences de la transpiration, sans altérer ou supprimer le processus biologique nécessaire de la sudation.

Certaines chaussures fermées, sans semelle ou textile adaptés, ne permettent pas l'évaporation, et la transpiration ainsi accumulée entraine une formation d'une couche humide et chaude à l'intérieur d'une chaussure ainsi que ses constituants, tels que, par exemple les semelles et le textile, formant un milieu très favorable à la prolifération de bactéries.

L'odeur désagréable issue de la transpiration est due à une accumulation de bactéries et à une macération de celles-ci à l'intérieur des chaussures.

La composition selon l'invention est destinée à être utilisée à l'intérieur d'une chaussure, d'une semelle ou du textile de la chaussure destiné à habiller, à protéger, et à soutenir un pied.

De manière avantageuse, l'application de cette composition ne permet aucun contact avec la peau, permettant à celle-ci de réaliser le processus normal de sudation.

La composition selon l'invention est constituée d'au moins un biocide.

De préférence, la composition selon l'invention est constituée essentiellement d'au moins un biocide.

On entend par « essentiellement », une quantité de substance entrant majoritairement dans la formulation de la composition selon l'invention, c'est-à-dire entre 40% et 98% en poids.

On entend par « biocide » tout produit destiné à assurer la protection des êtres humains, des animaux domestiques, des végétaux et de certains biens ou produits contre les organismes nuisibles, nocifs ou gênants.

Les biocides sont des substances actives ou des préparations contenant une ou plusieurs substances actives destinées à détruire, repousser ou rendre inoffensifs les organismes nuisibles, à en prévenir l'action ou à les combattre de toute autre manière, par une action chimique ou biologique.

En l'occurrence, le biocide utilisé dans la composition selon l'invention est, de préférence, un acide borique ou acide orthoborique ou acide boracique.

En effet, l'acide borique est un antiseptique faible et bactériostatique. Il est peu irritant pour la peau et les muqueuses.

De manière alternative, il peut être prévu que cette composition soit constituée d'une association d'au moins un biocide avec au moins un autre principe actif, de type connu et possédant, entre autres des propriétés antibactériennes, antiseptiques et antifongistatiques.

Les antibactériens, comme les antiseptiques et les antifongiques peuvent être bactéricides ou bactériostatiques.

Dans cette description, on entend par les termes « antifongiques» et « antibactériens », une action nocive uniquement sur l'inhibition de la croissance et de la reproduction, respectivement, des champignons et/ou moisissures et des bactéries, appelée également, respectivement, une action « fongistatique » et « bactériostatique ».

Par exemple, une substance active telle que de la sauge peut favorablement compléter l'action antiseptique et bactériostatique de la composition selon l'invention.

La composition selon l'invention comprend au moins une substance fongistatique et/ou odorante telle que de la lavande, par exemple.

Il peut être prévu d'ajouter au moins un agent absorbant et adsorbant tel que, par exemple, du kaolin ou/et ou de l'argile et/ou du yucca et/ou tout autre agent de ce type connu en soi, afin de renforcer la propriété absorbante et absorbante de la composition selon l'invention.

Cette composition peut être constituée d'autres d'excipients naturels ou synthétiques ou de mélange d'excipients de type connu en soi en fonction de la formulation, de la texture et du conditionnement finals souhaités.

A titre d'exemple non limitatif, la composition selon l'invention peut être une poudre et contenir environ de 40% à 98% d'acide borique, de 1.5% à 5.5% de kaolin, de 0.2% à 0.5% de lavande, de 0.05% à 0.2% de sauge et environ de 0.05% à 0.2% d'argile.

La composition, selon l'invention, possédant une action bactériostatique, fongistatique et antiseptique, absorbe et/ou adsorbe l'humidité présente notamment dans les chaussures, les semelles, et les textiles des chaussures par assèchement, et ce dès son application.

Ainsi, cette composition permet de supprimer et de prévenir l'apparition de macération des bactéries, des champignons et d'autres micro-organismes et donc empêcher les mauvaises odeurs résultantes, sans supprimer ou limiter la transpiration.
Préférentiellement, la composition, selon l'invention, se présente sous forme de poudre. Celle-ci est conditionnée sous la forme d'une boîte saupoudreuse recouverte d'une capsule, ou sous la forme d'un sachet hermétique permettant ainsi le saupoudrage de l'intérieur d'une chaussure, et plus particulièrement, de la semelle ou du textile de la chaussure.

Aussi, ladite composition peut être sous la forme de gel, de crème, de spray, de vaporisateur, de lotion, de stick ou de semelle ou de toute autre forme apte à pouvoir être appliquée à l'intérieur d'une chaussure, d'une semelle ou d'un textile de la chaussure.

La composition selon l'invention est utilisable pour tous types de chaussures tels que, par exemple, les chaussures de ville, de marche, de travail, de sécurité ou de sport.

## Revendications

1. Composition antibactérienne et antifongique, absorbant l'humidité des chaussures provenant de la transpiration des pieds, **caractérisée en ce qu'**elle est constituée d'au moins un biocide.

2. Composition selon la revendication 1, **caractérisée en ce que** ledit au moins un biocide comprend des fonctions antiseptiques, bactériostatiques et fongistatiques sans interférence avec le processus physiologique normal de sudation.

3. Composition selon l'une des revendications 1 ou 2, **caractérisée en ce que** ledit au moins un biocide est un acide borique ou acide orthoborique ou acide boracique.

4. Composition selon l'une des revendications précédentes, **caractérisée en ce qu'**elle est constituée d'au moins un autre principe actif.

5. Composition selon l'une des revendications précédentes, **caractérisée en ce qu'**elle contient au moins un agent absorbant et/ou adsorbant.

6. Composition selon l'une des revendications précédentes, **caractérisée en ce qu'**elle comprend au moins une substance odorante.

7. Composition selon l'une des revendications précédentes, **caractérisée en ce qu'**elle est constituée d'environ de 40 à 98 % d'acide borique, de 1.5 à 5.5% de kaolin, de 0.2 à 0.5% de lavande, de 0.05 à 0.2% de sauge et environ de 0.05 % à 0.2 % d'argile.

8. Composition selon l'une des revendications précédentes, **caractérisée en ce qu'**elle est sous forme de poudre.

9. Composition selon la revendication précédente, **caractérisée en ce qu'**elle est conditionnée dans une boîte saupoudreuse ou dans un sachet.

10. Composition selon l'une des revendications 1 à 7, **caractérisée en ce qu'**elle est sous forme de gel ou de crème ou de spray ou de vaporisateur ou de lotion ou de stick ou de semelle.
